(19) Europäisches Patentamt
European Patent Office
Office européen des brevets

(11) Publication number: 0 008 658
B1

(12) EUROPEAN PATENT SPECIFICATION

(45) Date of publication of patent specification: 01.12.82

(21) Application number: 79102618.0

(22) Date of filing: 24.07.79

(51) Int. Cl.[3]: C 07 C 101/72, C 07 C 101/28, C 07 D 209/20, C 07 D 233/64, A 61 K 31/195, A 61 K 31/405, A 61 K 31/415

# (54) Alpha-ethinyl-alpha-aminoacids and their esters, and pharmaceutical compositions containing them.

(30) Priority: 24.07.78 US 927439

(43) Date of publication of application:
19.03.80 Bulletin 80/6

(45) Publication of the grant of the patent:
01.12.82 Bulletin 82/48

(84) Designated Contracting States:
AT BE CH DE FR GB IT LU NL SE

(56) References cited:
EP - A - 0 000 035
DE - A - 2 827 824
US - A - 3 079 345
US - A - 4 103 089

TETRAHEDRON LETTERS NO. 32 (1977) + Pages 2745—2748 +
TETRAHEDRON LETTERS NO. 41 (1977) + Pages 3689—3692 +
BIOCHEMISTRY, 15 (1976) +Pages 3070—3076 +

(73) Proprietor: MERCK & CO. INC.
126, East Lincoln Avenue P.O. Box 2000
Rahway, New Jersey 07065 (US)

(72) Inventor: Taub, David
54 Wistar Avenue
Metuchen, N.J. 08840 (US)
Inventor: Patchett, Arthur Allan
1090 Minisink Way
Westfield, N.J. 07090 (US)

(74) Representative: Abitz, Walter, Dr.-Ing. et al,
Abitz, Morf, Gritschneder P.O. Box 86 01 09
D-8000 München 86 (DE)

EP 0 008 658 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Alpha-ethinyl-alpha-aminoacids and their esters, and pharmaceutical compositions containing them.

## Background of the Invention

The present invention concerns certain α-ethynyl-α-amino acids and esters.

α-Ethynyl-3,4-dihydroxyphenyl- alanine is disclosed in Tetrahedron Letters No. 32, p 2745—2748 (1977) and No. 41, p 3689—3692 (1977). An ethynyl amino acid is also disclosed in U.S. 3,079,345. A β-ethynyl-α-amino acid is described in Biochemistry *15*, 3070—76 (1976).

Novel α-ethynyl-α-amino acids and esters have been discovered; these compounds have pharmaceutical activity.

## Summary of the Invention

α-Ethynyl-α-amino-β-substituted propionic acids and esters.

## Description of the Preferred Embodiments

The present invention relates to compounds having the formula

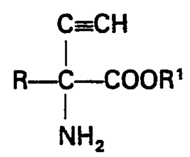

(I)

and pharmaceutically acceptable salts thereof wherein R is

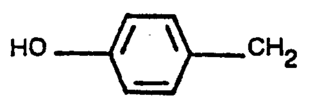
,
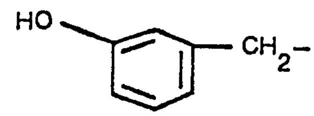
,
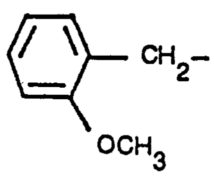
,
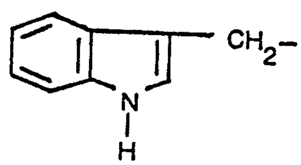
,
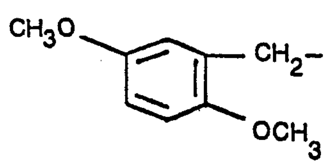
,
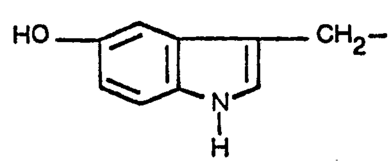
,
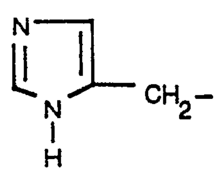

or $H_2N—(CH_2)_2—CH_2—$ and $R^1$ is H or $C_1—C_{18}$alkyl

The pharmaceutically acceptable salts are the acid addition salts of the Formula I compounds. Suitable acids include organic as well as inorganic acids. Preferred salts are the hydrohalides such as the hydrochlorides, hydrobromides and hydroiodides. Most preferred salts are the hydrochlorides.

The compounds of Formula I have a chiral center and thus occur in optically active forms, i.e., optical isomers. These isomers are conventionally designated as D and L, d and l, (+) and (—), (S) and (R) or by a combination of these symbols. Where a compound name or formula does not indicate a specific isomer, all forms are included, i.e., the individual isomers, mixtures thereof and racemates of the optical isomers, the L-form is preferred.

$R^1$ may be H or a $C_1—C_{18}$alkyl group. Examples of suitable alkyl groups are octadecyl, 2-ethylhexyl, lauryl, hexyl, heptyl, isopropyl, methyl and the like. Preferred $R^1$ groups are H and $C_1—C_6$ hydrocarbon alkyl. Most preferred $R^1$ groups are H and ethyl.

One class of preferred R groups are the methoxyphenyl and the hydroxyphenyl groups, especially:

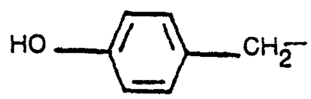 and 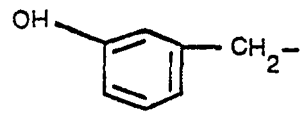

Another preferred R group is the imidazolyl moiety:

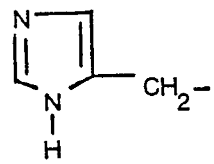

Illustrative examples of useful compounds are:

2-ethynyl-2-amino-3 (4-hydroxyphenyl) propionic acid,

2-ethynyl-2-amino-3 (3-indolyl) propionic acid,

2-ethynyl-2-amino-3 (2-methoxyphenyl) propionic acid,

2-ethynyl-2-amino-3 (5-hydroxyindol-3-yl) propionic acid,

2-ethynyl-2-amino-3 (2,5-dimethoxy-phenyl) propionic acid,

2-ethynyl-2-amino-3 (3-hydroxyphenyl) propionic acid octadecyl ester,

2-ethynyl-2-amino-3 (2-imidazolyl) propionic acid,

2-ethynyl-2-amino-5-aminopentanoic acid,

2-ethynyl-2-amino-3 (2-methoxyphenyl) propionic acid ethyl ester.

and the like.

The compounds of the present invention have pharmaceutical activity and exhibit enzyme inhibition[1]. Compounds of Formula I where R is said methoxy or hydroxy phenyl substituents have antihypertensive activity. Thus, they are useful for treating hypertension in human beings alone or in combination with carbidopa.

For treating hypertension, these compounds may be administered to the hypertensive patient orally or parenterally. Suitable conventional dosage forms are used such as tablets, troches, capsules, liquid formulations, e.g., solutions, dispersions, emulsions and the like. These pharmaceutical compositions may contain conventional pharmaceutically acceptable compounding ingredients, i.e., diluents, carriers, etc. and conventional preparative procedures are used.

The daily antihypertensive dosage may be varied as required. In general, a daily dosage range for the hypertensive patient is about 50 mg. to about 5000 mg., preferably from about 100 mg. to about 3500 mg. and more preferably from about 250 mg. to about 1500 mg.

Compounds of Formula I where R is the imidazolyl moiety are pharmaceutically useful as gastric secretion inhibitors or as antihistamine antiallergics. Effective daily dosages for these compounds may be varied as necessary. Daily dosage may range, e.g., from about 100 mg. to about 3000 mg. Dosage forms, suitable for the administration mode, e.g., oral, insufflation, parenteral, are used.

Compounds of Formula I where R is $NH_2—CH_2—CH_2—CH_2—$ are useful as antitumor agents. Mode of administration of these compounds may be oral or parenteral. Effective daily dosage may be varied and may range from about 100 mg. to about 5000 mg. Suitable dosage forms prepared using conventional procedures and compounding ingredients i.e., diluents, carriers etc., are used.

The present compound may be prepared by any convenient process. The processes described in the aforesaid Tetrahedron Letters references may be used.

The following examples illustrate preparation of representative compounds of Formula I. All temperatures are in degrees Celsius.

---

[1] This attribute makes the compounds useful as biochemical reagents.

## Example 1

4-Benzyloxybenzyl bromide *1*

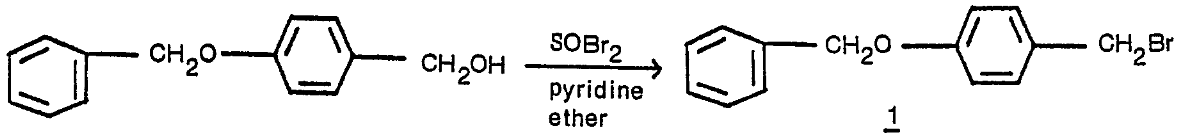


To a stirred solution of 5.54 g (20 mmol) of 4-benzyloxybenzyl alcohol and 2 ml of pyridine in 40 ml of ether at 0° is added dropwise a solution of thionyl bromide 4.4 g (2.1 mmol) in 20 ml of ether. The mixture is stirred at room temperature for 2 hours, cold water is added and the mixture is extracted with ether. The ether layer is washed thoroughly with water, saturated sodium chloride solution, dried over sodium sulfate and concentrated to dryness to give 4-benzyloxybenzyl bromide *1*.

## Example 2

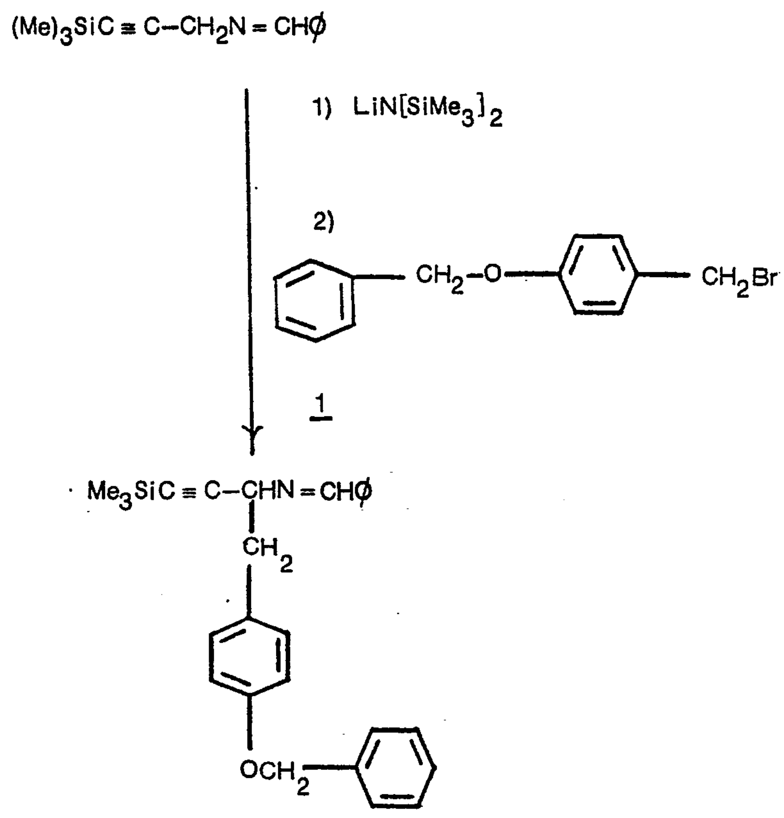


2

3-(4-Benzyloxybenzyl) 1-trimethylsilyl-N-benzylidene-3-aminoprop-1-yne *2*.

To a stirred solution of hexamethyldisilazane (330 mg; 2.05 mmol) in 4 ml tetrahydrofuran at −70° under nitrogen is added dropwise 1.25 ml of 1.6M n-butyl lithium in hexane (2.0 mmol). After 15 minutes 1-trimethylsilyl-N-benzylidene-3-aminoprop-1-yne (430 mg; 2.0 mmol) in 4.5 ml of tetrahydrofuran is added over 4 minutes. After an additional 10 minutes, 4-benzyloxybenzyl bromide *1* (554 mg; 2.0 mmol) in 4 ml tetrahydrofuran is added over 4 minutes. The mixture is stirred at −70° for 4 hours. It is then added to 20 ml of saturated aqueous ammonium chloride, extracted with ether and the ether extract washed with saturated aqueous sodium chloride and dried over magnesium sulfate. Concentration to dryness yields alkylation product *2* as an orange colored oil, m.s. $M^+$—411.

Example 3

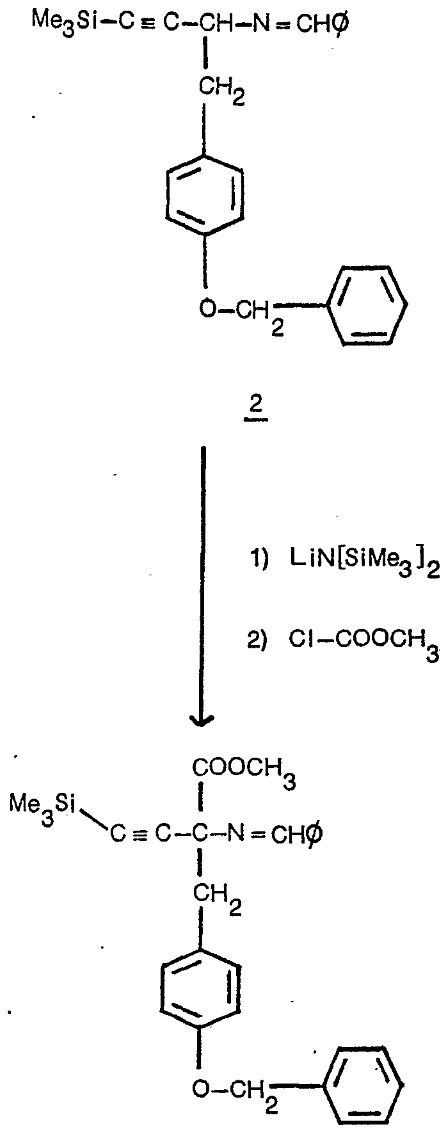


2

3

3-Carbomethoxy-3(4-benzyloxybenzyl) 1-trimethylsilyl-N-benzylidene-3-aminprop-1-yne *3*

To a stirred solution of hex methyl-disilazane (210 mg; 1.3 mmol) in 3 ml tetrahydrofuran at −70° under nitrogen is added dropwise 0.75 ml of 1.6M n-butyl lithium (1.2 mmol). After 5 minutes alkylation product *2* (500 mg; 1.2 mmol) in 6 ml of tetrahydrofuran is added over 6 minutes. After 5 minutes, a solution of methyl chlor formate (245 mg; 2.6 mmol) in 2 ml of tetrahydrofuran is added over 2 minutes and the reaction mixture is stirred at −70° for 20 hours. The mixture is added to 25 ml of saturated aqueous ammonium chloride, extracted with ether, the ether extract washed with saturated ammonium chloride, saturated sodium chloride, dried over magnesium sulfate and concentrated to dryness to give *3* as a viscous orange oil (600 mg) m.s. $M^+$—469.

Example 4

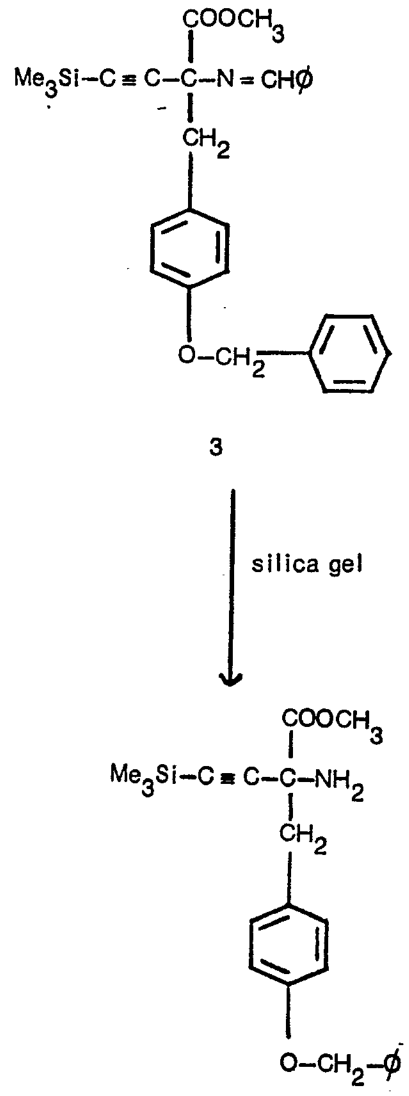


3

4

3-Carbomethoxy-3-(4-benzyloxybenzyl)-1-trimethylsilyl-3-aminoprop-1-yne *4*

Chromatography of crude *3* (600 mg) on 25 g of silica gel H eluting with chloroform –2% acetonitrile leads to hydrolysis of the Schiff base protecting group to give the free amine *4*, m.s., $M^{+}$—381.

Example 5

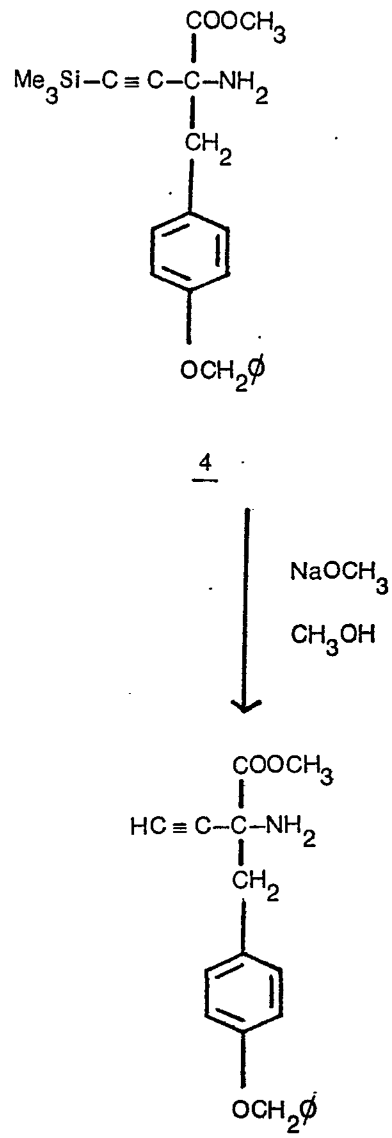


4

5

3-Carbomethoxy-3-(4-benzyloxybenzyl)-3-aminoprop-1-yne *5*

To a solution of intermediate *4* (760 mg; 2.0 mmol) in 8 ml of methanol under nitrogen at 20° is added 1.5 ml of 1.75M methanolic sodium methoxide. After 30 minutes at room temperature methylene chloride and water are added. The aqueous layer is washed with methylene chloride at the combined organic phase washed with saturated salt solution, dried over sodium sulfate and concentrated to dryness to give *5*, nmr ($CDCl_3$) γ 2.46 (s,1H), 2.70 (broad S,2H), 3.14 (s,2H), 3.78 (s,3H), 5.04 (s,2H) 6.8—7.5 (m,9H).

Example 6

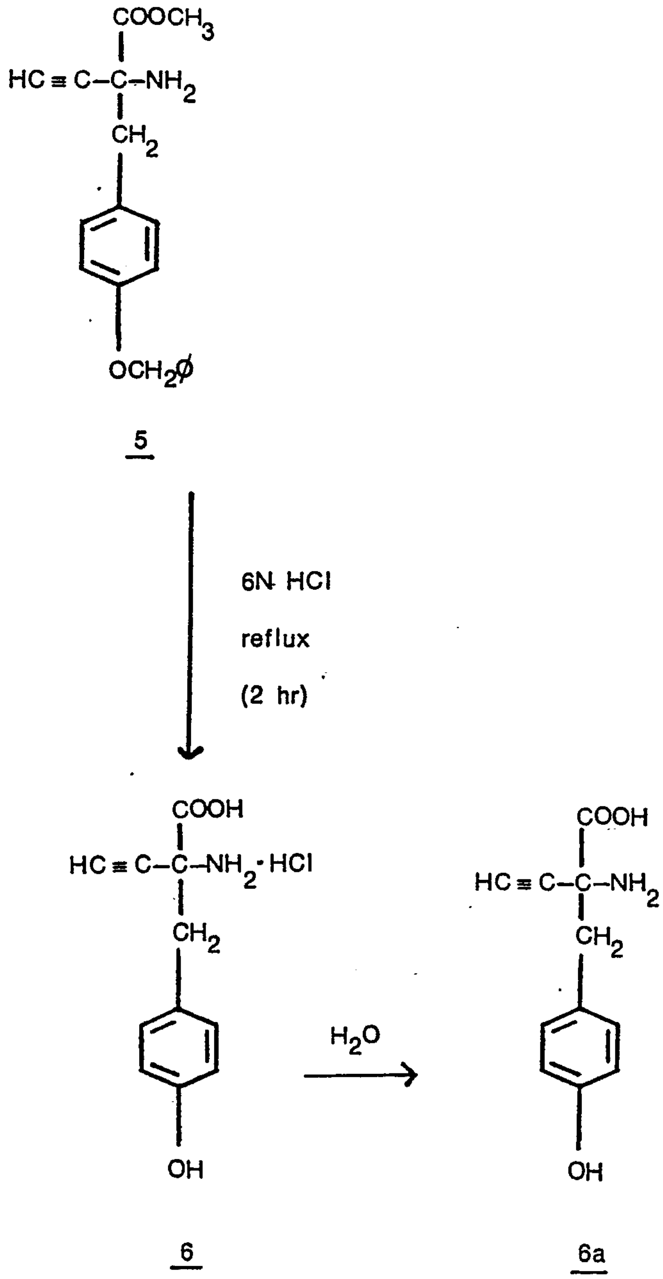


5

6

6a

$\alpha$-Ethynyltyrosine hydrochloride *6* and $\alpha$-ethynyltyrosine *6a*

To Compound *5* (465 mg.; 1.5 mmol) is added 30 ml of 6N hydrochloric acid and the mixture is stirred and refluxed for 2 hours. The mixture is cooled and extracted with methylene chloride. The aqueous acid phase is treated with charcoal, filtered and concentrated to dryness to give $\alpha$-ethynyl tyrosine hydrochloride *6*. The latter, when dissolved in the minimum amount of water and chilled, gives crystals of $\alpha$-ethynyltyrosine *6A*. m.p. 214—215°.

Example 7

Preparation of $\alpha$-Ethynyl-m-tyrosine *7*

In a similar manner starting with 3-benzyloxybenzyl alcholol, and following the procedures of Examples 1—6, is produced $\alpha$-ethynyl-m-tyrosine hydrochloride *7a*.

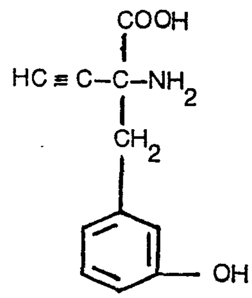

7

The salt *7a* is converted to the free acid *7* by dissolving 100 mg in 1 ml of water and 2 ml of acetone, adding 2 ml of propylene oxide and after 18 hours concentrating to dryness.

Example 8

α-Ethynyl-β-(-2 methoxyphenyl)-alanine *9*

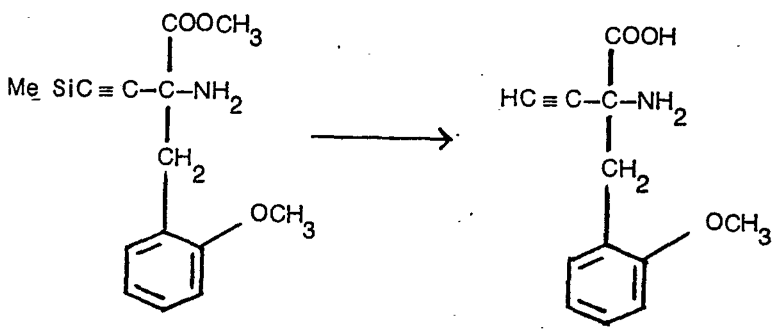

8 9

Following the procedures of Examples 1—4 and starting with 2-methoxybenzyl alcohol, there is produced 3-carbomethoxy-3-(2-methoxybenzyl)-1-trimethysilyl-3-aminoprop-1-yne *8*. To a stirred solution of the latter (610 mg; 2 mmol) in 10 ml of methanol is added 8 ml of 1N sodium hydroxide dropwise over 5 minutes. The mixture is stirred 2 hours at room temperature, neutralized with dilute hydrochloric acid and concentrated to dryness. The residue is purified by ion exchange chromatography on Dowex 50WX4 (Trade Mark) resin on the acid cycle to give α-ethynyl-β-(2-methoxy-phenyl) alanine HCl converted to 9 by treatment with propylene oxide as in Example 7.

Example 9

α-Ethynyl-β-(2,5-dimethoxyphenyl)alanine *10*

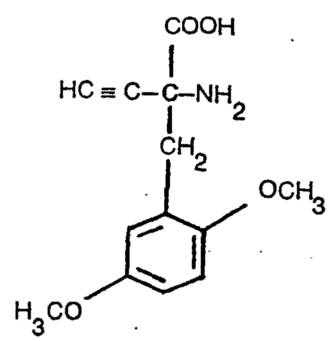

10

Following the procedures of Example 8 and starting with 2,5-dimethoxy-benzyl alcohol there is produced α-ethynyl-β-(2,5-dimethoxyphenyl)alanine *10*.

Example 10

α-Ethynylhistidine *11*

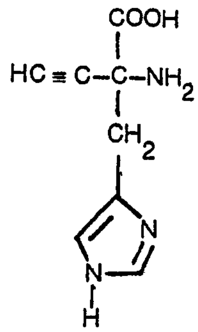

Starting with N-p-toluenesulfonyl-4-acetoxymethyl-imidazole and following the procedure of Example 2 except that the reaction mixture is kept 4 hours at –70° and 2 hours at 0°; and following the procedures for Examples 3—6, except that the final acid hydrolysis is performed with 12N hydrochloric acid for 4 hours, there is obtained *11* as a mixture of hydrochloride and p-toluenesulfonate salts. The mixture is all converted into the dihydrochloride by chromatography over Dowex 50WX4 (Trade Mark), eluting with 2N hydrochloric acid. Conversion to the free acid is accomplished by the propylene oxide procedure of Example *8*.

Example 11

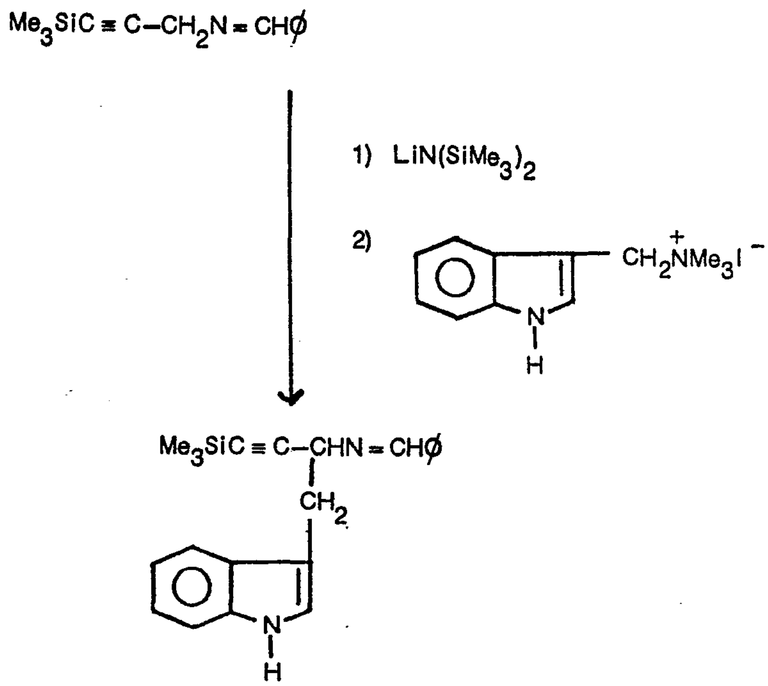


12

3-(3-indolylmethyl)-1-trimethylsilyl-N-benzylidene-3-aminoprop-1-yne *12*

Using the procedure of Example *2* and gramine methiodide (636 mg; 2 mmol) as alkylating agent there is obtained compound *12*.

Example 12

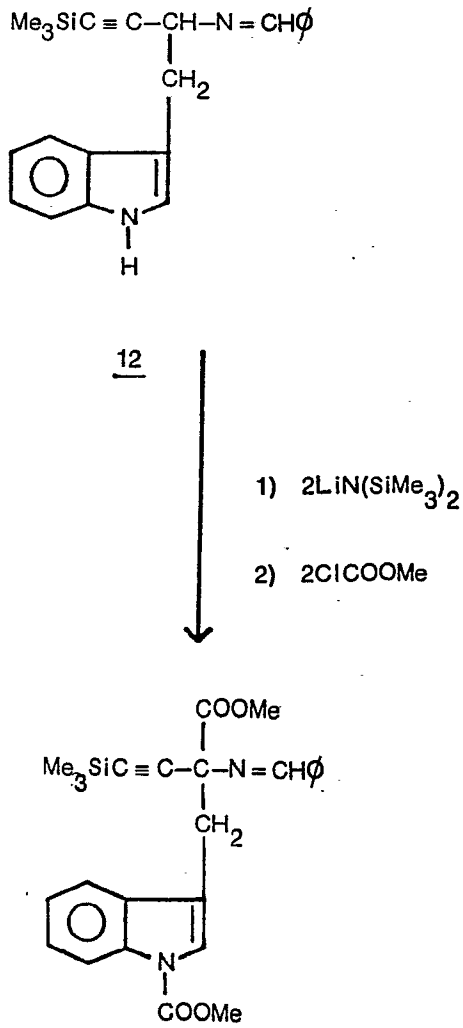


12

13

3-Carbomethoxy-3-(3-N-carbomethoxyindolylmethyl) 1-trimethylsilyl-N-benzylidene-3-aminoprop-1-yne *13*

Using the procedure of Example 3 on *12* in place of *2*, except that the molar quantities of hexamethyl-disilazane, n-butyl lithium and methyl chloroformate are doubled, there is obtained Compound *13*.

Example 13

α-Ethynyltryptophan *14*

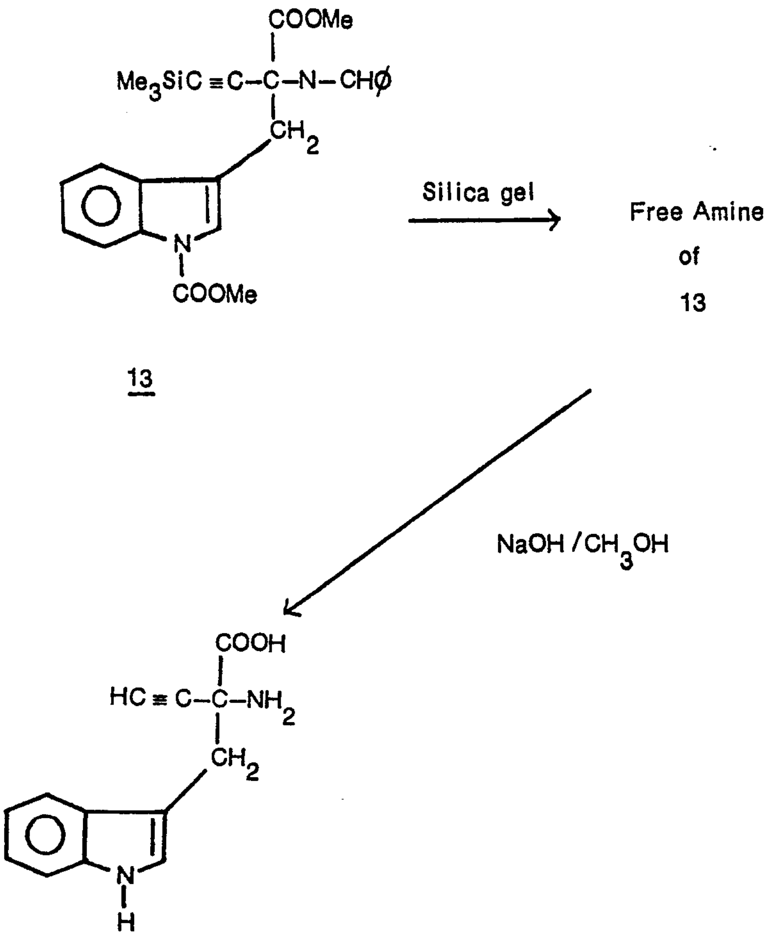


13

14

Chromatography of crude *13* on silica gel H, as described in Example 4, yields the corresponding free amine. To a stirred solution of the latter (370 mg; 1 mmol) in 10 ml of methanol is added 10 ml of 2N aqueous sodium hydroxide and the mixture is refluxed for 2 hours under nitrogen. The reaction mixture is then neutralized with dilute hydrochloric acid and concentrated to dryness under reduced pressure. The residue is taken up in hot water, the mixture treated with activated charcoal and the filtrate purified by ion exchange chromatography on Dowex 50WX4 (Trade Mark) resin ($H^+$ form). Following initial elution with water, elution with 1M ammonium acetate followed by desalting of the pertinent fractions by passage through BioRad Ag 11 A-8 (Trade Mark) ion-retardation resin, elution with water and concentration to dryness of the pertinent fractions yields pure *14*.

Example 14

5-Methoxymethoxy-2-nitrobenzaldehyde *15*

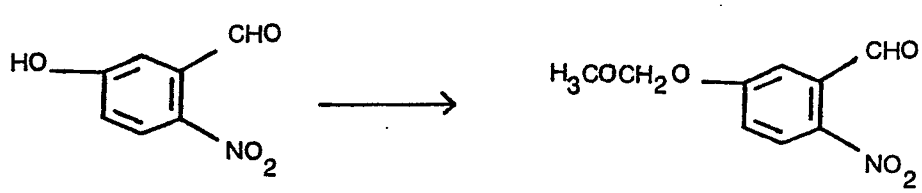

15

To a stirred mixture of 5-hydroxy-2-nitrobenzaldehyde (16.7 g; 0.10 mol) and sodium methoxide (5.4 g; 0.1 mol) in toluene (100 ml) at 0° is added dropwise chloromethylmethyl ether (8.1 g; 0.1 mol)

over 15 minutes and the mixture is stirred at room temperature 1 hour and refluxed 1 hour. The mixture is cooled, washed with water, 5% aqueous sodium hydroxide, saturated aqueous sodium chloride, dried over sodium sulfate and concentrated to dryness under reduced pressure to give *15*.

## Example 15

5-Methoxymethoxyindole *16*

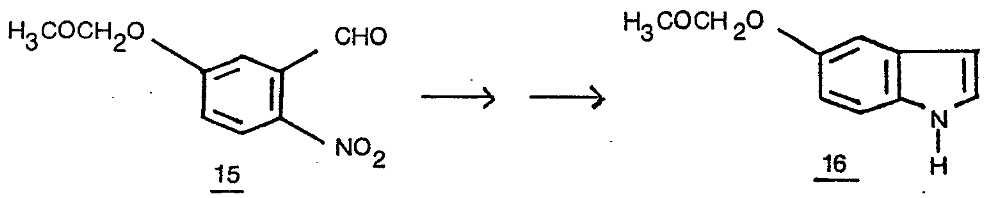

15 16

5-Methoxymethoxyindole *16* is prepared from 5-methoxymethoxy-2-nitrobenzaldehyde *15* by the procedure of A. Ek and B. Witkap [J.A.C.S. *76* 5579 (1954)] utilized for the preparation of 5-benzyloxy-indole from 5-benzyloxy-2-nitrobenzaldehyde.

## Example 16

5-Methoxymethoxygramine methiodide *17*

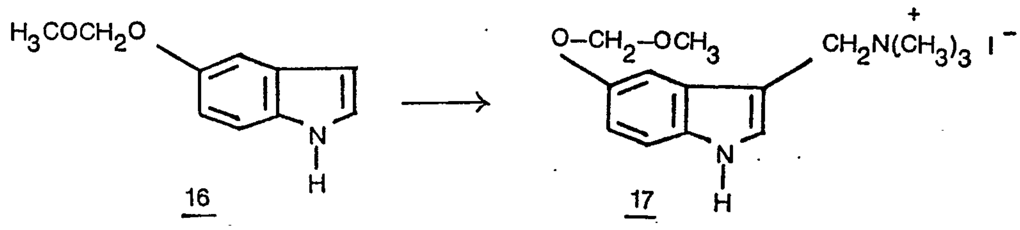

16 17

To a stirred mixture at 0° of 13.5 g (0.10 mol) of 35% aqueous dimethylamine and 7.2 g (0.12 mol) of acetic acid is added 7.6 g of 40% formaldehyde (0.10 mol) followed by 17 g (0.10 mol) of *16* in 30 ml of dioxane. The mixture is stirred at 0° 1 hour and 18 hours at 20°. Sodium hydroxide solution (100 ml of 5%) is added and the mixture is cooled to 0°. The precipitated 5-methoxymethoxygramine is filtered, washed with water and dried under vacuum. It is converted to the methiodide *17* by adding 10 g in portions with stirring to 100 ml of methyl iodide at 5°. After 10 hours at 5°, methiodide *17* is filtered, washed with ether and dried under vacuum.

## Example 17

5-Methoxymethoxy-$\alpha$-ethynyltryptophan *18*

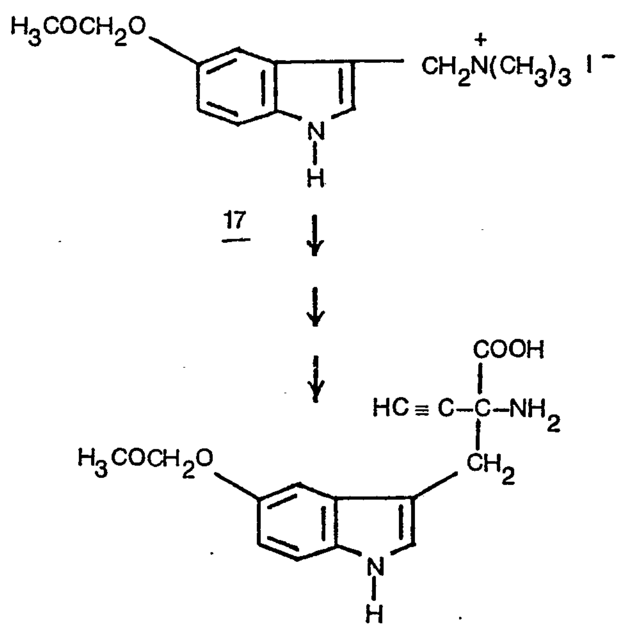

17

18

By the procedures of Examples 11—13, 5-methoxymethoxy gramine methiodide *17* is converted into 5-methoxymethoxy-$\alpha$-ethynyl tryptophan *18*.

Example 18

5-Hydroxy-$\alpha$-ethynyltryptophan *19*

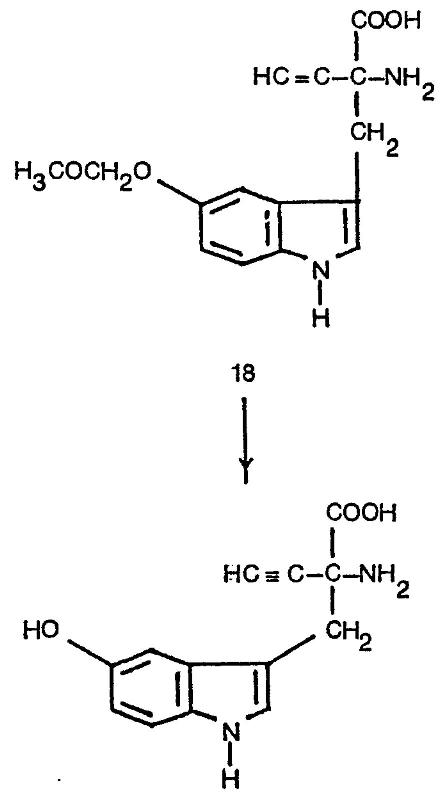

18

19

A solution of intermediate *18* (200 mg) in 20 ml of water and 0.2 ml of 2N hydrochloric acid is stirred at 80° for 5 minutes. The mixture is cooled, just neutralized with aqueous sodium bicarbonate and concentrated to dryness under vacuum. Desalting by passage of the residue in water through Bio Rad Ag 11 A-8 (Trade Mark) ion-retardation resin, elution with water and concentration of the pertinent fractions to dryness under vacuum yields pure *19*.

Where $R^1$ in Formula I is an alkyl group, the compound is prepared by conventional esterification of the corresponding compound where R is H as illustrated by the following equation:

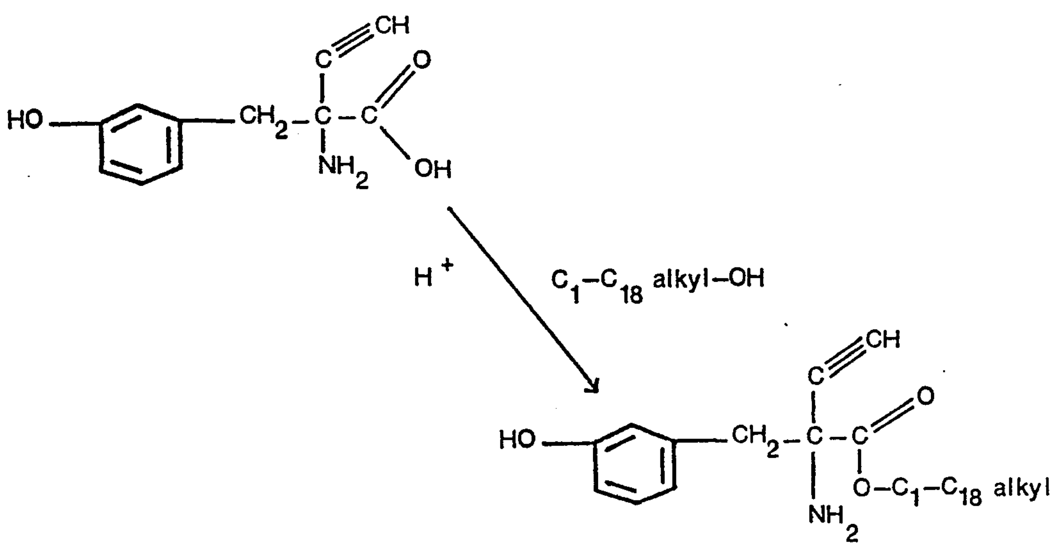

The pharmaceutically acceptable salts of the present compounds may be obtained by treatment of the Formula I free base with an appropriate acid under suitable conditions.

The products obtained in the examples are racemates. These racemates may be resolved using conventional techniques, e.g., by treating the racemate with a suitable optically active acid in a suitable solvent and recovering the individual amino acid isomer from the resultant salt.

Claims to the invention follow.

**Claims for the Contracting States: BE CH DE FR GB IT LU NL SE**

1. Compounds having the formula

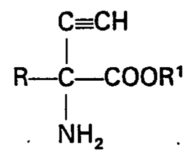

and pharmaceutically acceptable salts thereof wherein R is

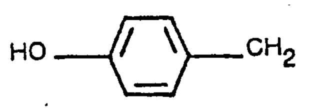 , 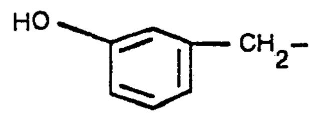 ,

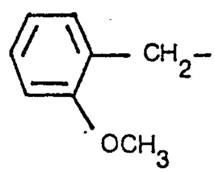 , 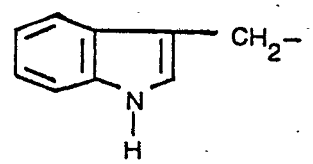 ,

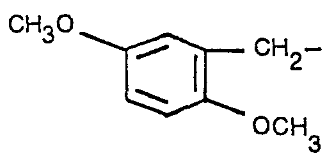 , 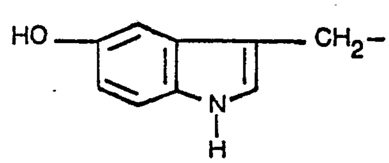 ,

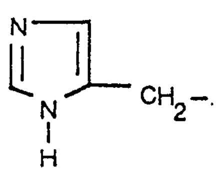 or $H_2N—(CH_2)_2—CH_2—$ and $R^1$ is H or $C_1—C_{18}$alkyl.

2. Compounds of Claim 1 wherein $R^1$ is $C_1—C_6$alkyl.
3. Compounds of Claim 1 wherein $R^1$ is H.
4. Compounds of Claim 1 having the L-configuration.
5. Compounds of Claim 1 wherein R is

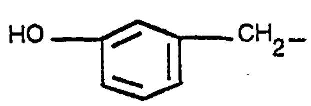 or 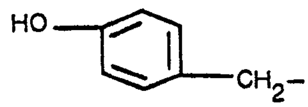

6. Compounds of Claim 1 wherein R is

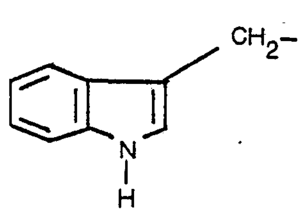 or 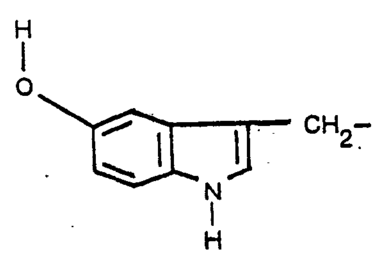

7. Compounds of Claim 1 wherein R is

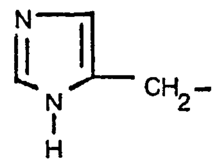

8. Compounds of Claims 5, 6 or 7 wherein $R^1$ is H.

9. The L-isomer of a compound of Claim 8.

10. A pharmaceutical composition containing a therapeutically effective amount of a Claim 1 compound.

**Claims for the Contracting State: AT**

1. A process for preparing compounds having the formula

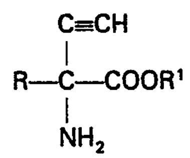

(I)

wherein R is

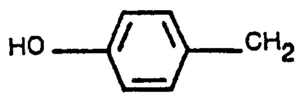 ,
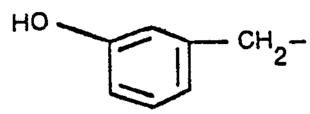 ,
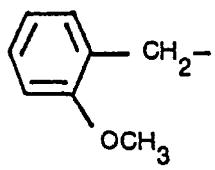 ,
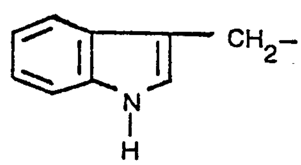 ,
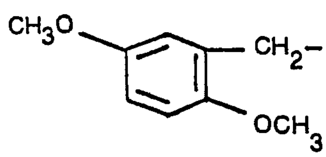 ,
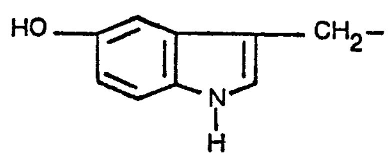 ,
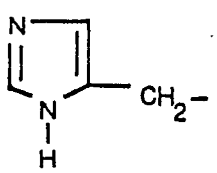 or $H_2N—(CH_2)_2—CH_2—$

and $R^1$ is H or $C_1—C_{18}$alkyl which comprises

(a) hydrolysis of a compound of the formula

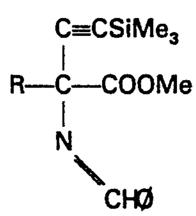

wherein when R contains OH or —NH— substituent the substituent may be protected, to prepare compound I where $R^1$ is H and

(b) conventionally alkylating the product from (a) with a suitable $C_1—C_{18}$ alkylating agent to obtain compound I where $R^1$ is $C_1—C_{18}$alkyl.

2. A process (a) of Claim 1 wherein R is

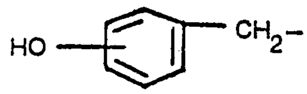

and the OH protecting group is

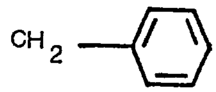

3. A process (a) of Claim 1 wherein R is

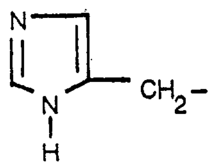 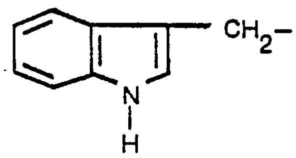 or 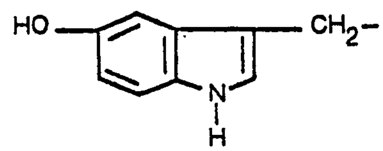

and the NH protecting group is COOMe and the OH protecting group is $CH_2—CO—CH_3$.

**Revendications pour les Etats contractants: BE CH DE FR GB IT LU NL SE**

1. Composés de formule

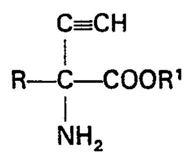

et leurs sels pharmaceutiquement acceptables, où R représente

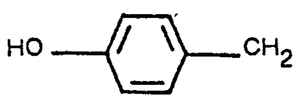 , 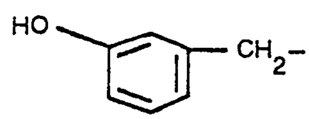 ,

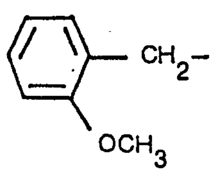 , 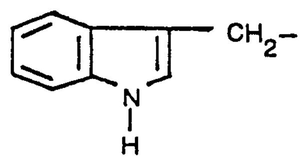 ,

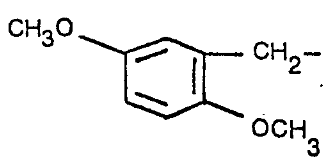 , 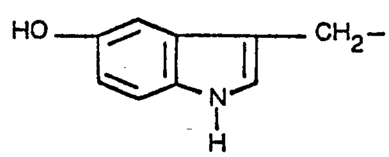 ,

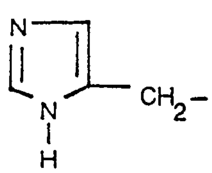 or $H_2N—(CH_2)_2—CH_2—$

et où $R^1$ est H ou un alcoyle en $C_1$ à $C_{18}$.

2. Composés de la revendication 1 où $R^1$ est un alcoyle en $C_1$ à $C_6$.

3. Composés de la revendication 1 où $R^1$ est H.

4. Composés de la revendication 1 ayant la configuration L.

5. Composés de la revendication 1 où R est

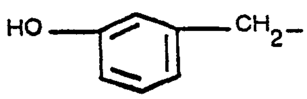 ou 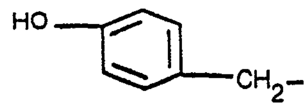

6. Composés de la revendication 1 où R est

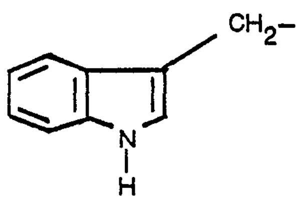 ou 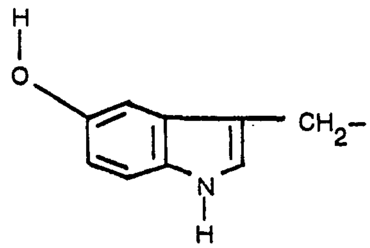

7. Composés de la revendication 1 où R est

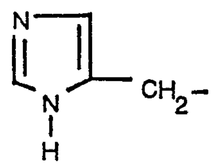

8. Composés des revendications 5, 6 ou 7 où $R^1$ est H.

9. Isomère L d'un composés de la revendication 8.

10. Composition pharmaceutique contenant une quantité thérapeutiquement efficace d'un composé de la revendication 1.

**Revendications pour l'Etat contractant: AT**

1. Procédé de préparation de composés de formule

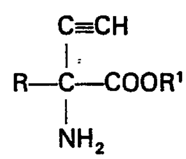 (I)

où R est

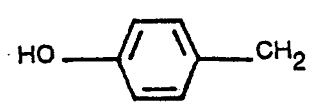 , 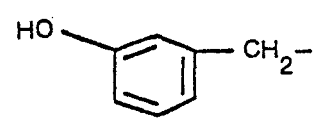 ,

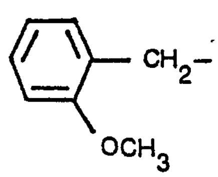 , 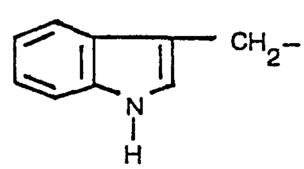 ,

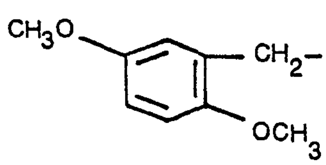 , 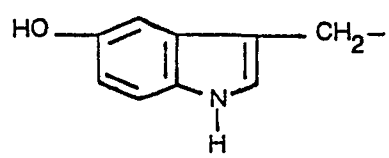 ,

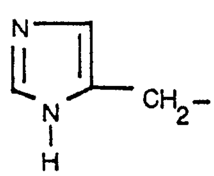 ou $H_2N—(CH_2)_2—CH_2—$

et $R^1$ est H ou un alcoyle en $C_1$ à $C_{18}$, dans lequel

(a) on hydrolyse un composé de formule

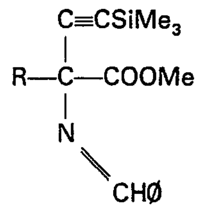

où lorsque R contient un substituant OH ou —NH—, le substituant peut être protégé, pour préparer un composé I où R est H et

(b) on alcoyle de façon classique le produit de a) avec un agent alcoylant en $C_1$ à $C_{18}$ approprié pour obtenir un composé I où $R^1$ est un alcoyle en $C_1$ à $C_{18}$.

2. Procédé a) de la revendication 1 où R est

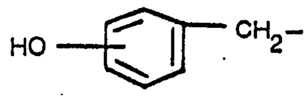

et le groupe protecteur d'OH est

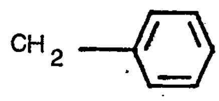

3. Procédé a) de la revendication 1 où R est

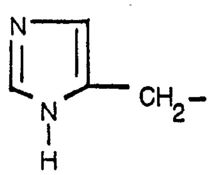
,
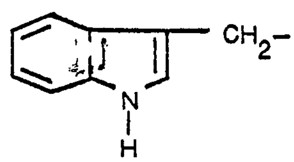
ou
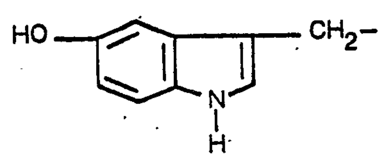

et le groupe protecteur de NH est COOMe et le groupe protecteur d'OH est $CH_2$—CO—$CH_3$.

**Patentansprüche für die Vertragsstaaten: BE CH DE FR GB IT LU NL SE**

1. Verbindungen der Formel

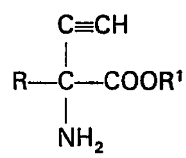

und pharmazeutisch annehmbare Salze davon, worin R

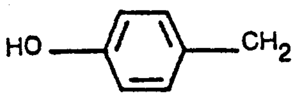 , 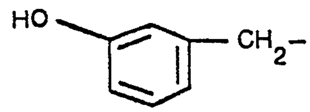 ,

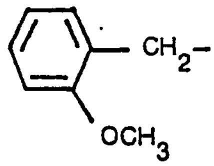 , 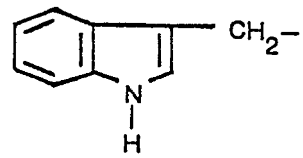 ,

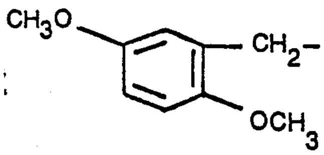 , 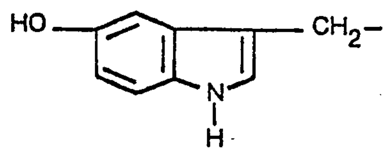 ,

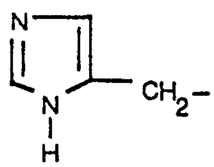 oder $H_2N—(CH_2)_2—CH_2—$

ist und $R^1$ H oder $C_1$—$C_{18}$-Alkyl ist.

2. Verbindungen nach Anspruch 1, worin $R^1$ $C_1$—$C_6$-Alkyl ist.

3. Verbindungen nach Anspruch 1, worin $R^1$ H ist.

4. Verbindungen nach Anspruch 1, welche die L-Konfiguration haben.

5. Verbindungen nach Anspruch 1, worin R

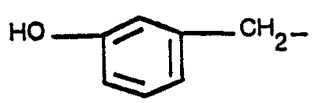 oder 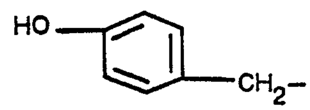

ist.

6. Verbindungen nach Anspruch 1, worin R

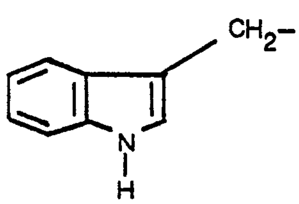 oder 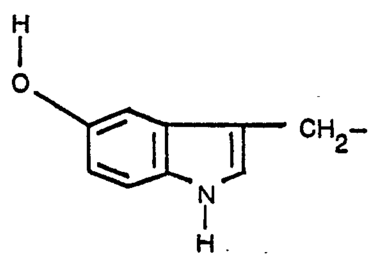

ist.

7. Verbindungen nach Anspruch 1, worin R

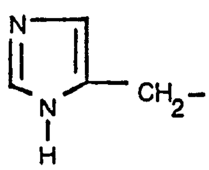

ist.

8. Verbindungen nach Anspruch 5, 6 oder 7, worin $R^1$ H ist.

9. Das L-Isomer einer Verbindung des Anspruchs 8.

10. Pharmazeutische Zusammensetzung, enthaltend eine therapeutisch wirksame Menge einer Verbindung nach Anspruch 1.

**Patentansprüche für den Vertragsstaat: AT**

1. Ein Verfahren zur Herstellung von Verbindungen der Formel

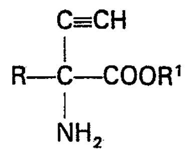

(I)

worin R

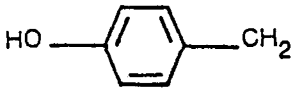 , 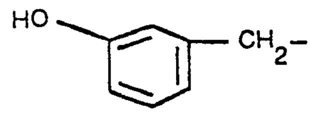 ,

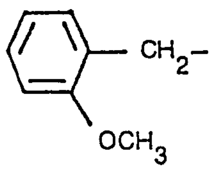 , 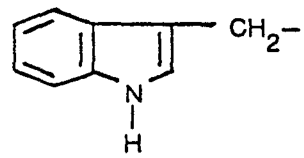 ,

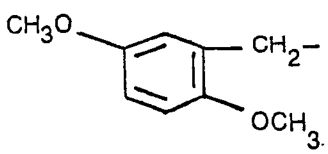 , 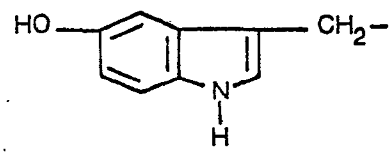 ,

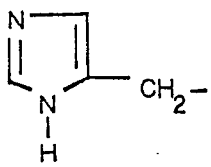 oder $H_2N—(CH_2)_2—CH_2—$

ist und $R^1$ H oder $C_1—C_{18}$-Alkyl ist, umfassend

(a) Hydrolyse einer Verbindung der Formel

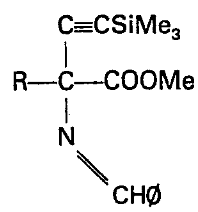

worin, falls R OH oder —NH-Substituenten enthält, der Substituent geschützt sein kann, zur Herstellung einer Verbindung I, worin $R^1$ H ist und

(b) übliche Alkylierung des Produktes aus (a) mit einem geeigneten $C_1—C_{18}$-Alkylierungsmittel zur Gewinnung einer Verbindung I, worin $R^1$ $C_1—C_{18}$-Alkyl ist.

2. Ein Verfahren (a) nach Anspruch 1, worin R

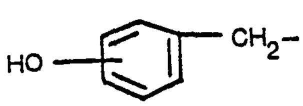

ist und die OH-Schutzgruppe

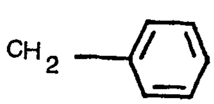

ist.

3. Ein Verfahren (a) nach Anspruch 1, worin R

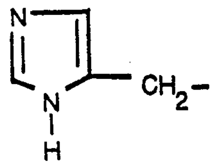 , 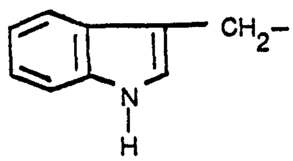 oder 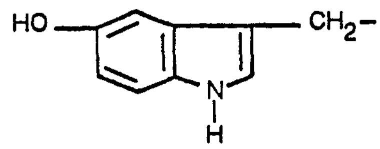

ist und die NH-Schutzgruppe COOMe ist und die OH-Schutzgruppe $CH_2—CO—CH_3$ ist.